(19) **Europäisches Patentamt**
**European Patent Office**
**Office européen des brevets**

(11) **EP 3 405 041 B1**

(12) **EUROPEAN PATENT SPECIFICATION**

(45) Date of publication and mention
of the grant of the patent:
**26.05.2021 Bulletin 2021/21**

(21) Application number: **17741157.6**

(22) Date of filing: **17.01.2017**

(51) Int Cl.:
*A61K 35/742* *(2015.01)*       *A61K 35/747* *(2015.01)*
*A61K 35/74* *(2015.01)*       *A61K 36/064* *(2006.01)*
*A23C 9/123* *(2006.01)*       *A23C 9/127* *(2006.01)*
*C12N 1/20* *(2006.01)*       *A23L 33/135* *(2016.01)*
*A23L 33/14* *(2016.01)*

(86) International application number:
**PCT/IB2017/050232**

(87) International publication number:
**WO 2017/125845 (27.07.2017 Gazette 2017/30)**

(54) **A LIQUID PROBIOTIC COMPOSITION STABLE AT AMBIENT TEMPERATURE**

BEI UMGEBUNGSTEMPERATUR STABILE FLÜSSIGE PROBIOTISCHE ZUSAMMENSETZUNG

COMPOSITION PROBIOTIQUE LIQUIDE STABLE À TEMPÉRATURE AMBIANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.01.2016 IN 201621001880**

(43) Date of publication of application:
**28.11.2018 Bulletin 2018/48**

(73) Proprietor: **Synergia Life Sciences Pvt. Ltd
Mumbai 400022 (IN)**

(72) Inventors:
• **MEHTA, Dilip
Mumbai 400006 (IN)**
• **DE SOUZA, Anselm
Mumbai 400080 (IN)**

(74) Representative: **Kasche, André
Kasche & Partner AG
Bühlstrasse 1
8125 Zollikerberg / Zürich (CH)**

(56) References cited:
**EP-A2- 0 203 586       WO-A1-2008/039531
WO-A1-2016/198440       WO-A2-2015/061789
US-A1- 2005 042 207       US-A1- 2010 086 646
US-A1- 2015 079 234       US-A9- 2015 196 609
US-B2- 8 097 245**

• **YEO SIOK-KOON ET AL: "Effect of prebiotics on
viability and growth characteristics of probiotics
in soymilk", JOURNAL OF THE SCIENCE OF
FOOD AND AGRICULTURE, vol. 90, no. 2, 30
January 2010 (2010-01-30), pages 267-275,
XP002614574,**

## Description

### Related Application

This application claims priority from provisional application number 201621001880 dated 19th January 2016 filed with Mumbai Patent Office (India).

### Field of Invention

The present invention relates to stable liquid composition according to the claims. Disclosed are stable liquid syrup compositions comprising probiotic bacteria, retaining their cellular viability at ambient storage conditions for at least two years. More particularly the disclosure relates to stable liquid syrup compositions comprising probiotic bacteria belonging to the genera *bacillus / lactobacillus* which retain at least 75 % of the activity when stored at ambient storage conditions for subsequent use as probiotic syrup, food, food supplement, and pharmaceutical compositions.

### Background of the Invention

Probiotics can be termed as "live microorganisms" which when consumed in adequate amounts confer a health effect on the host. Probiotics today find applications in diverse fields such as food, meal replacements, dietary supplements, nutraceuticals, over the counter drugs as well as prescription drugs. The most widely marketed probiotics are dairy products and probiotic-fortified food. The products have to be stored at low temperatures to maintain the probiotic activity and have a relatively shorter shelf life. Further Probiotics can grow in these products using carbon and nitrogen sources and ferment the products giving rise to offensive odour to the products. In other fields tablets, capsules, and sachets containing the bacteria in freeze-dried form are more common. Probiotics play an important role in immunological, digestive and respiratory functions and could have a significant effect in alleviating disease conditions especially in children.

Probiotics therapy has been successfully tried with antibiotics associated diarrhoea as well as rotavirus diarrhoea, inflammatory disease and bowel syndromes, mucosal immunity and various other indications such as urogenital tract disorder, bacterial vaginosis, yeast vaginitis and also to maintain a healthy gut flora (as a prophylactic) in healthy individuals Most probiotic formulations are solid oral dosage forms. These are the probiotic powders (originally freeze dried or spray dried) mixed with excipients and formulated as per requirement to tablets, capsules, powder sachets, etc.

The probiotic requirement varies depending on the strain, product type, intended application and stability. According to Council for Agricultural Science and Technology the products often do not meet label claims with regard to the numbers and types of viable microbes present in the product, and do not deliver the quantity that needs to be consumed for a health benefit. The Council further recommends that manufacturers label the genus, species, and strain for each probiotic in a product, along with the number of viable cells of each probiotic strain that will remain up to the end of shelf-life. It is imperative that the products deliver requisite dose throughout the duration of the shelf-life and exhibit minimal batch to batch variation.

Accordingly, the product should amongst other aspects, comply with the following label requirements:

1) Genus and species identification, according to scientific nomenclature 2) Designate strain 3) Indicate viable count at the end of shelf-life 4) Specify storage conditions 5) Specify recommended dose based on the physiological effect desired.

Most probiotic formulations are provided as solid oral dosage forms. In general there is a need for a palatable liquid probiotic formulation which can be stored at ambient conditions without significant loss in probiotic activity over the recommended shelf life of the product. However until now there are no liquid probiotic formulations available in the market, which are palatable and can be stored at ambient storage conditions without significant loss in probiotic activity over the recommended shelf life of the product. This is because the Probiotics can grow using carbon and nitrogen sources in the formulation causing gas from fermentation and also giving rise to offensive odour and taste. The objective of the present invention is to meet this long felt need. Moreover, liquid probiotic formulations as compared to tablets will have better compliance in paediatric and geriatric populations.

Preparation of liquid probiotic formulations is highly desirable since elaborate and expensive processes such as freeze drying and microencapsulation used in the preparation of solid formulations are eliminated. The products are easy to administer. However preparation of liquid probiotic formulations which retain their cellular viability under ambient storage conditions over extended time periods to provide long shelf life to the product, poses following challenges.

1. The bacteria will start growing in the liquid media leading to metabolite production at ambient temperature.

2. It is known that the sugars act as substrates and enhance the metabolite production at ambient temperatures.

3. Gas producing probiotics will bulge the containers and produce offensive odours and taste.

4. Metabolite production will result in death of the probiotic strain and lower counts.

Shelf life of such a formulation will be very short at ambient temperatures.

## Discussion of the Related Art

"Enterogermina" produced and marketed by Sanofi is a liquid oral suspension of *Bacillus clausi* spores containing 2 billion spores in 5 ml saline. While this is a liquid suspension (formulation) it is not palatable and appealing in terms of taste, especially to children and more so to children with nausea associated with antibiotic associated diarrhoea. WO2005017095 describes a liquid probiotic composition of *E. coli* M17. This is a suspension of culture free washed *E.coli* in 0.2 - 0.6% saline buffered to a pH of 6.5 - 6.8. The product needs to be stored between +2°C to +8 °C.

Patent application US20120171329 refers to a probiotic fruit juice drink composed of probiotic bacteria and a gas formation reducer chosen from acelora, pomegranate, cranberry, argnia, blackcurrant, blackthorn or elderberry extract only. The probiotic bacteria herein is chosen from *Lactobacillus* species, especially *Lactobacillus plantarum.* The product needs to be stored between 4 - 8°C. This composition is then further added to the other juices which are required to be made gas free. This limits the scope of invention to the specified gas reducer juice combinations only.

Patent application US20100086646 relates to fresh plant juice and or milk based food product comprising live probiotics and a dietary protonated weak acid with a pH between 3-4 which prevented false taste and gas in the product.

As cited in US20130295226, pH and temperature affect the organoleptic properties of the food product and the survival of the probiotic. These inventors used an acid adapted strain of *L. casei* and *L. paracasei* to overcome the above problems of pH and temperature limitations. This method is therefore restricted to specially adapted strains.

WO2008/039531 discloses probiotic compositions that have enhanced stability under various storage conditions through the addition of agents and excipients such as sugar alcohols and/or phytonutrients.

## Sumary of the Invention

It has been now found that liquid compositions comprising probiotic bacteria of the genera bacillus / lactobacillus, are stable at ambient storage conditions in the presence of sweeteners such as sugars which constitute at least 60 % by weight of the liquid compositions comprising probiotic bacteria.

The present invention relates to stable liquid compositions comprising probiotic bacteria of the genera bacillus/lactobacillus, hydrophilic vehicles, solubilizers, pH modifiers, buffers, viscosity modifiers, preservatives, stabilizers and sweeteners. The said compositions retain their cellular viability under ambient storage conditions for at least two years as measured by the spores count test. The said compositions retain their cellular viability under accelerated aging test conditions for at least six months as measured by the spores count test. The compositions of the present invention are stable for use as probiotic syrup, in food, food supplement, and pharmaceutical applications.

Also disclosed is a liquid composition comprising yeast and at least 60% sweetener by weight, and one more or more excipients selected from the group consisting of hydrophilic vehicles, solubilizer, pH modifier, buffer, viscosity modifier, preservatives, and stabilizers, wherein the said probiotic bacteria retains at least 75% of its cellular viability of the original value as measured by the spore count. The yeast of the disclosure is preferably saccharomyces boulardii.

## Detailed Description of the Invention

The objectives of the present invention are accomplished and the shortcomings associated with the probiotic compositions of the prior art are overcome by the present invention as described below in the preferred embodiments.

The present invention encompasses liquid compositions of probiotic bacteria that are stable at ambient conditions. The bacteria of the invention is selected from the group consisting of genera *bacillus.*

In an embodiment the percentage of sweetener in the formulation is preferably 60%, 70% or 80%.

In an embodiment, the formulation of the invention comprises at least 60% sweetener, which does not promote the growth of bacterial spores.

The probiotic compositions of the present disclosure may have pH in the range 3.5 to 6.0.

In yet another embodiment the present invention provides probiotic compositions comprising 1) one or more *Bacillus* species in spore form, 2) a hydrophilic vehicle 3) a solubilizer which is a surfactant 4) pH modifiers and buffering agents 5) viscosity modifying agents 6) preservatives 7) stabilizers and 8) sweeteners

In yet another embodiment, the present invention also provides probiotic compositions comprising 1) one or more Lactobacillus species, 2) a hydrophilic vehicle 3) a solubilizer which is a surfactant 4) pH modifiers and buffering agents. 5) Viscosity modifying agents 6) preservatives 7) stabilizers and 8) sweeteners.

Further disclosed are probiotic compositions which when stored at ambient storage conditions for 24 months do not promote E coli growth.

Further disclosed are probiotic compositions wherein the pH of the composition when stored at ambient storage conditions for 24 months does not decrease by more than 0.1.

In another embodiment, the invention provides a liquid composition comprising probiotic bacteria which belongs to the genus *Bacillus.*

In another embodiment the invention provides a liquid composition comprising probiotic bacteria selected from *Bacillus subtilus, Bacillus clausi and Bacillus coagulans.*

In another embodiment the invention provides a liquid composition comprising a probiotic bacteria which belongs to the genus Lactobacillus.

In another embodiment the invention provides a liquid composition comprising a probiotic bacteria selected from *lactobacillus acidophilus* and *lactobacillus casei*

Further disclosed is a liquid composition comprising probiotic bacteria which is a mixture of probiotic bacteria selected from the genus bacillus and lactobacillus.

Further disclosed is a liquid composition comprising a probiotic bacteria and a sweetener.

Further disclosed is a liquid composition comprising a probiotic bacteria and a sweetener wherein the sweetener is a sugar.

In another embodiment the invention provides a liquid composition comprising a probiotic bacteria and a sugar selected from sucrose, glucose, and fructose.

In another embodiment the invention provides a liquid composition comprising a probiotic bacteria and a sugar wherein the sugar is selected from a mixture of sucrose, glucose, and fructose.

Further disclosed is a liquid composition comprising a probiotic bacteria and a sweetener wherein the sweetener is a mixture of sugar and sugar alcohol.

In another disclosure, the liquid compositions comprising probiotic bacteria, which are not stable in the presence of sugar alcohol alone, are stable in the presence of sugar alcohols and sugars.

In another embodiment the invention provides a liquid composition comprising a probiotic bacteria and a mixture of sugar and sugar alcohol, wherein the sugar alcohol is selected from mannitol, sorbitol and xylitol.

In another embodiment the invention provides a liquid composition comprising a probiotic bacteria and a sugar and sugar alcohol wherein the ratio of sugar to sugar alcohol ranges from 0.85 to 2.25.

In a preferred embodiment the compositions of the invention retain cellular viability under ambient storage conditions for at least two years for use as probiotic syrup in food, food supplement and pharmaceutical composition, particularly for health care.

The bacteriostatic preservative in the formulation is selected from the group consisting of bronidiol, a combination of methyl paraben and propyl paraben, sodium salt of parabens. Preferably, the preservative is bronidiol.

The present disclosure describes probiotic compositions having pH in the range 3.5 to 6.0.

Citric acid serves as the pH modifier in the instant formulation.

The present disclosure also envisages a liquid composition comprising yeast and at least 60% sweetener by weight, and one more or more excipients selected from the group consisting of hydrophilic vehicles, solubilizer, pH modifier, buffer, viscosity modifier, preservatives, and stabilizers, wherein the said probiotic bacteria retains at least 75% of its cellular viability of the original value as measured by the spore count. The yeast of the disclosure is preferably saccharomyces boulardii.

The foregoing descriptions of specific embodiments of the present invention have been presented for purposes of illustration and description only. They are not intended to be exhaustive or to limit the present invention to the precise forms disclosed, and obviously many modifications and variations are possible in light of the above teaching.

**Example 1**

**Method of preparation of Probiotic Composition**

The compositions of the present invention are prepared as per the procedure described below: Water is heated in a vessel to 70°C. Vehicle and sweetener are added with stirring. After a homogenous solution is formed, the same is filtered to clarify and cooled to 45 °C. The probiotic powder is added with stirring to form a homogenous solution. All other components of the formulation except the bacteriostatic preservative are added at this stage. Finally the bacteriostatic preservative, for example, Bronidiol is added and the volume of the composition is made up to 1000 ml using requisite quantity of water.

4

**Example 2**

**Method for the determination of spores count**

1. Take 1 gram of sample and suspend in 99 ml of saline +0.5 % Tween 80 in an Erlenmeyer flask (This is 100 times dilution)
2. Keep it on a rotary shaker (200 rpm) at 37°C for 30 mins.
3. Transfer 0.5 ml from the above flask to 4.5 ml of saline and carry out serial dilutions up to $10^9$
4. Surface spread 0.1 ml of the last 3 (10, $10^8$, $10^9$) dilutions in duplicate on sterile Luria Bertani agar or Soybean casein Digest media (SCDM) agar plate using sterile spreader.
5. Incubate the plates at 37°C and count the number of colony forming units.

$$\text{Spore count CFU/gm} = \text{Number of colonies x Dilution factor x volume of sample used}$$

| For example | If $10^7$ dilution plate gave 10 colonies, then |
|---|---|
| Spore count CFU/gm | $10 \times 10^7 \times 10 = 1.0 \times 10^9$ cfu/ml or $5.0 \times 10^9$ cfu/5ml |

Media composition

| 1) | Diluent | 0.85 gm |
|---|---|---|
| | Sodium chloride | |
| | Tween 80 | 0.5 gm |
| | Distilled water | 100 ml |
| 2) | Phenol red dextrose broth | |
| | Phenol red dextrose broth (Hi media MO56) | 2.1 gm |
| | Distilled water | 100 ml |
| 3) | Soya bean casein digest medium (SCDM) | |
| | Soya bean casein digest medium broth ( Himedia MO11) | 3 gm |
| | Agar powder (Himedia -RM026) | 2 gm |
| | Distilled water | 100 ml |
| 4) | Luria Bertani agar | |
| | Tryptone (Casein hydrolysate) | 1gm |
| | Yeast extract | 0.5 gm |
| | Sodium chloride | 0.5 gm |
| | Distilled water | 100 ml |
| | pH | 7.5 |
| | Agar powder (Himedia -RM026) | 2gm |

For all spore count measurements to evaluate stability following conditions were used .

**Ambient storage conditions**

Temperature 30°C$\pm$2 °C, Humidity 75 % RH$\pm$5% RH
RH = Relative Humidity

**Storage conditions for accelerated aging test**

Temperature 40°$\pm$2 °C, Humidity 65 % RH$\pm$5% RH

**Example 3**

**Formulation of the Probiotic Composition**

| Sucrose | 600 gm |
|---|---|
| B. subtilis | $2 \times 10^9 / 5\,ml$ |
| Flavour strawberry | 5 gm |
| Colour FDC Red | 0.2 gm |
| Bronidiol | 1 gm |
| Water q. s. | 1000 ml |

Results of accelerated stability test

| Months | 0 | 1 | 2 | 3 | 6 |
|---|---|---|---|---|---|
| Spore count $10^9 / 5ml$ | 3.8 | 3.1 | 2.85 | 4.1 | 3.7 |

Results of stability test under ambient storage conditions

| Months | 0 | 1 | 2 | 3 | 6 | 12 | 18 | 24 |
|---|---|---|---|---|---|---|---|---|
| Spore count $10^9 / 5ml$ | 2.7 | 2.4 | 3.6 | 2.6 | 3.1 | 2.5 | 2.2 | 2.6 |
| pH | 3.5 | 3.5 | 3.4 | 3.6 | 3.5 | 3.4 | 3.5 | 3.6 |

The comparative results show that results of stability test under ambient storage conditions are adequately simulated by the results of the accelerated stability test.

**Example 4**

**Formulation of the Probiotic Composition with change in bacterial concentration**

| Sucrose | 600 gm |
|---|---|
| B. subtilis | $0.2 \times 10^9 / 5\,ml$ |
| Flavour strawberry | 5 gm |
| Colour FDC Red | 0.2 gm |
| Bronidiol | 1 gm |
| Water q. s. | 1000 ml |

| Sucrose | 600 gm |
|---|---|
| B. subtilis | $10 \times 10^9 / 5\,ml$ |
| Flavour strawberry | 5 gm |
| Colour FDC Red | 0.2 gm |
| Bronidiol | 1 gm |

(continued)

| Water q. s. | 1000 ml |
| --- | --- |

## Example 5

**Formulation of the Probiotic Composition with change in sweetener**

| High fructose corn syrup | 780 gm |
| --- | --- |
| B. subtilis | $2 \times 10^9$ / 5 ml |
| Flavour strawberry | 5 gm |
| Colour FDC Red | 0.2 gm |
| Bronidiol | 1 gm |
| Water q. s. | 1000 ml |

| Sucrose + High fructose corn syrup | 300 gm + 390 gm |
| --- | --- |
| B. subtilis | $2 \times 10^9$ / 5 ml |
| Flavour strawberry | 5 gm |
| Colour FDC Red | 0.2 gm |
| Bronidiol | 1 gm |
| Water q. s. | 1000 ml |

## Example 6

| Sucrose | 600 gm |
| --- | --- |
| Sorbitol | 150 gm |
| B. subtilis | $2 \times 10^9$ / 5 ml |
| Flavour strawberry | 5 gm |
| Colour FDC Red | 0.2 gm |
| Bronidiol | 1 gm |
| Water q.s. | 1000 ml |

| Sucrose | 600 gm |
| --- | --- |
| Glycerol | 50 gm |
| B. subtilis | $2 \times 10^9$ / 5 ml |
| Flavour strawberry | 5 gm |
| Colour FDC Red | 0.2 gm |
| Bronidiol | 1 gm |
| Water q.s. | 1000 ml |

### Example 7

**Formulation of the Probiotic Composition with pH modifier**

| | |
|---|---|
| Sucrose | 600 gm |
| B. subtilis | $2x10^9$ / 5 ml |
| Flavour strawberry | 5 gm |
| Colour FDC Red | 0.2 gm |
| Bronidiol | 1 gm |
| Water q. s. | 1000 ml |
| Citric acid | 0.1 % |

### Example 8

**Formulation of the Probiotic Composition with change in preservative**

| | |
|---|---|
| Sucrose | 600 gm |
| B. subtilis | $2x10^9$ / 5 ml |
| Flavour strawberry | 5 gm |
| Colour FDC Red | 0.2 gm |
| Methyl Paraben + Propyl Paraben sodium salt | 0.075 % + 0.075 % |
| Water q. s. | 1000 ml |

| | |
|---|---|
| Sucrose | 600 gm |
| B. subtilis | $2x10^9$/ 5 ml |
| Flavour strawberry | 5 gm |
| Colour FDC Red | 0.2 gm |
| Sodium Benzoate | 0.1 % |
| Water q. s. | 1000 ml |

### Example 9

**Formulation of the Probiotic Composition with a thickener**

| | |
|---|---|
| Sucrose | 600 gm |
| B. subtilis | $2x10^9$ / 5ml |
| Flavour strawberry | 5 gm |
| Colour FDC Red | 0.2 gm |
| Bronidiol | 1 gm |

(continued)

| | |
|---|---|
| Water q. s. | 1000 ml |
| Methyl cellulose | 0.5 % |

| | |
|---|---|
| Sucrose | 600 gm |
| B. subtilis | $2x10^9$/ 5 ml |
| Flavour strawberry | 5 gm |
| Colour FDC Red | 0.2 gm |
| Bronidiol | 1 gm |
| Water q. s. | 1000 ml |
| Carboxy methyl cellulose | 1 % |

In the following examples only the contents of the probiotic bacteria and the sugar / sugar alcohol are mentioned. Other ingredients are as per example 3

**Example 10**

| | |
|---|---|
| Sucrose | 300 gm |
| Sorbitol | 350 gm |

Results of accelerated stability test

| Months | 0 | 1 | 2 | 3 | 6 |
|---|---|---|---|---|---|
| Spore count $10^9$/ 5ml | 2.7 | 2.4 | 3.6 | 2.6 | 3.1 |

**Example 11**

| | |
|---|---|
| Sorbitol | 650 gm |

Composition not stable

**Example 12**

| | |
|---|---|
| Sucrose | 250 gm |
| Mannitol | 400gm |

Results of accelerated stability test

| Months | 0 | 1 | 2 | 3 | 6 |
|---|---|---|---|---|---|
| Spore count $10^9$/ 5ml | 3.1 | 2.4 | 2.9 | 3.6 | 3.4 |

**Example 13**

| Mannitol | 650 gm |
|----------|--------|

Composition not stable

**Example 14**

| Sucrose | 550 gm |
|---------|--------|
| Glycerol | 100 gm |
| B. subtilis | $6.47 \times 10^9$/ 5 ml |

Results of accelerated stability test

| Months | 0 | 1 | 2 | 3 | 6 |
|--------|---|---|---|---|---|
| Spore count $10^9$/5ml | 3.8 | 3.1 | 2.85 | 4.1 | 3.7 |

**Example 15**

| Glycerol | 650 gm |
|----------|--------|
| B. subtilis | $6.2 \times 10^9$/ 5 ml |

Composition not stable

**Example 16**

| Sucrose | 350 gm |
|---------|--------|
| Glucose | 100 gm |
| Sorbitol | 200 gm |
| B. subtilis | $6.4 \times 10^9$/ 5 ml |

Results of accelerated stability test

| Months | 0 | 1 | 2 | 3 | 6 |
|--------|---|---|---|---|---|
| Spore count $10^9$/ 5ml | 2.7 | 2.4 | 3.6 | 2.6 | 3.1 |

**Example 17**

| Sucrose | 250 gm |
|---------|--------|
| Glucose | 250 gm |

(continued)

| Glycerol | 150 gm |
|---|---|
| B. subtilis | 5.8 x $10^9$/ 5 ml |

Results of accelerated stability test

| Months | 0 | 1 | 2 | 3 | 6 |
|---|---|---|---|---|---|
| Spore count $10^9$ / 5ml | 3.1 | 2.4 | 2.9 | 3.6 | 3.4 |

## Example 18

In this and all subsequent examples the liquid probiotic composition as in example 3 is used . The bacteria species and content is varied as indicated
Species Bacillus clausi : Content $3.8 \times 10^9$ / 5ml
Results of accelerated stability test

| Months | 0 | 1 | 2 | 3 | 6 |
|---|---|---|---|---|---|
| Spore count $10^9$ / 5ml | 3.8 | 3.5 | 2.9 | 3.1 | 3.2 |
| pH | 3.6 | 3.5 | 3.6 | 3.6 | 3.6 |

Results of stability test under ambient conditions

| Months | 0 | 1 | 2 | 3 | 6 | 12 | 18 | 24 |
|---|---|---|---|---|---|---|---|---|
| Spore count $10^9$ / 5ml | 3.8 | 3.1 | 3.3 | 3.1 | 3.2 | 3.6 | 3.7 | 3.5 |
| pH | 3.6 | 3.6 | 3.5 | 3.6 | 3.5 | 3.5 | 3.6 | 3.5 |

## Example 19

Species Bacillus coagulans : Content $2.4 \times 10^9$ / 5ml
Results of accelerated stability test

| Months | 0 | 1 | 2 | 3 | 6 |
|---|---|---|---|---|---|
| Spore count $10^9$ / 5ml | 2.4 | 2.2 | 2.1 | 2.4 | 2.2 |
| pH | 3.6 | 3.6 | 3.6 | 3.5 | 3.6 |

Results of stability test under ambient conditions

| Months | 0 | 1 | 2 | 3 | 6 | 12 | 18 | 24 |
|---|---|---|---|---|---|---|---|---|
| Spore count $10^9$ / 5ml | 2.4 | 2.2 | 2.1 | 2.4 | 2.2 | 2.6 | 2.1 | 2.5 |
| pH | 3.6 | 3.6 | 3.6 | 3.6 | 3.5 | 3.5 | 3.6 | 3.6 |

## Example 20

Species: Lactobacillus acidophilus Content $2.8 \times 10^9$ / 5ml
Results of accelerated stability test

| Months | 0 | 1 | 2 | 3 | 6 |
|---|---|---|---|---|---|
| Spore count $10^9$ / 5ml | 2.4 | 2.2 | 2.1 | 2.4 | 2.2 |

**Example 21**

Species: Bacillus subtilis Content 3.8x$10^9$ / 5ml + Lactobacillus casei Content 3.4 x$10^9$ / 5ml

Results of accelerated stability test

| Months | 0 | 1 | 2 | 3 | 6 |
|---|---|---|---|---|---|
| Bacillus subtilis Spore count $10^9$ / 5ml | 3.8 | 3.1 | 2.85 | 4.1 | 3.7 |
| Lactobacillus casei Spore count $10^9$ / 5ml | 3.4 | 3.2 | 3.1 | 3.6 | 3.1 |
| pH | 3.5 | 3.5 | 3.5 | 3.5 | 3.6 |

**Claims**

1.  A liquid composition comprising

    (a) probiotic bacteria selected from the species Bacillus subtilis, Bacillus clausi, Bacillus coagulans, Lactobacillus acidophilus and Lactobacillus casei,
    (b) at least 60% weight by volume of a sweetener selected from a sugar and a mixture of a sugar and sugar alcohol, and
    (c) a preservative,

    wherein said probiotic bacteria retains at least 75% of its cellular viability of the original value as measured by the spore count at ambient storage conditions for a period of at least 6 to 24 months,
    and wherein the sugar is selected from the group consisting of sucrose, glucose and fructose, and/or the sugar alcohol is mannitol, sorbitol, xylitol or a mixture thereof.

2.  The liquid composition according to claim 1 further comprising one more or more excipients selected from the group consisting of hydrophilic vehicles, solubilizer, pH modifier, buffer, viscosity modifier, and stabilizers.

3.  The liquid composition according to any of claims 1 or 2, wherein the spore count of the composition is in the range 0.2x$10^9$ cfu/5ml to 10x$10^9$ cfu/5ml.

4.  The liquid composition according to any of claims 1 to 3, wherein the sweetener is a mixture of sugar and sugar alcohol at ratio of sugar to sugar alcohol in the range of 0.85 to 2.25.

**Patentansprüche**

1.  Eine flüssige Zusammensetzung umfassend

    (a) probiotische Bakterien ausgewählt aus den Spezies Bacillus subtilis, Bacillus clausi, Bacillus coagulans, Lactobacillus acidophilus und Lactobacillus casei,
    (b) wenigstens 60 Gew.-% pro Volumen eines Süssungsmittels ausgewählt aus einem Zucker und einer Mischung aus einem Zucker und Zuckeralkohol, sowie
    (c) ein Konservierungsmittel,

    wobei die probiotischen Bakterien wenigstens 75% des ursprünglichen Wertes ihrer zellulären Entwicklungsfähigkeit beibehalten, der durch Sporenzählung bei Raumlagerungsbedingungen für eine Dauer von wenigstens 6 bis 24 Monate gemessen wird, und wobei der Zucker aus der Gruppe bestehend aus Sukrose, Glukose und Fruktose, ausgewählt wird, und/oder der Zuckeralkohol ist Mannitol, Sorbitol, Xylitol oder eine Mischung davon.

2.  Die flüssige Zusammensetzung gemäss Anspruch 1 zusätzlich umfassend einen oder mehrere Hilfsstoffe ausgewählt aus der Gruppe bestehend aus hydrophilen Trägerstoffen, Lösungsvermittlern, pH-modifizierenden Stoffen, Pufferstoffen, Viskositätsmodifikatoren und Stabilisatoren.

3. Die flüssige Zusammensetzung gemäss einem der Ansprüche 1 oder 2, wobei die Sporenzahl der Zusammnesetzung in dem Bereich von 0,2x10$^9$ cfu (Kolonie formende Einheiten) / 5 ml bis 10x10$^9$ cfu (Kolonie formende Einheiten) /5 ml liegt.

4. Die flüssige Zusammensetzung gemäss einem der Ansprüche 1 bis 3, wobei das Süssungsmittel eine Mischung aus einem Zucker und einem Zuckeralkohol in dem Verhältnis von Zucker zu Zuckeralkohol in dem Bereich von 0,85 bis 2,25 ist.

**Revendications**

1. Composition liquide comprenant :

(a) une bactérie probiotique choisie parmi les espèces Bacillus subtilis, Bacillus clausi, Bacillus coagulans, Lactobacillus acidophilus et Lactobacillus casei,
(b) au moins 60% de poids sur volume d'un édulcorant choisi parmi un sucre et un mélange d'un sucre et d'un alcool de sucre, et
(c) un conservateur,

dans laquelle ladite bactérie probiotique conserve au moins 75% de sa viabilité cellulaire de la valeur d'origine telle que mesurée par le comptage de spores à des conditions ambiantes de stockage pendant une période d'au moins 6 à 24 mois,
et dans laquelle le sucre est choisi dans le groupe constitué de saccharose, de glucose et de fructose et/ou l'alcool de sucre est le mannitol, le sorbitol, le xylitol ou un mélange de ceux-ci.

2. Composition liquide selon la revendication 1 comprenant en outre un excipient de plus ou plus choisis dans le groupe constitué de véhicules hydrophiles, solubilisant, modificateur de pH, tampon, modificateur de viscosité et stabilisants.

3. Composition liquide selon l'une quelconque des revendications 1 ou 2, dans laquelle le comptage de spores de la composition est dans l'intervalle de 0,2 x 10$^9$ cfu/5 ml à 10 x 10$^9$ cfu/5 ml.

4. Composition liquide selon l'une quelconque des revendications 1 à 3, dans laquelle l'édulcorant est un mélange de sucre et d'alcool de sucre à un rapport de sucre sur alcool de sucre dans l'intervalle de 0,85 à 2,25.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201621001880
- WO 2005017095 A
- US 20120171329 A
- US 20100086646 A
- US 20130295226 A
- WO 2008039531 A